# EUROPEAN PATENT APPLICATION

(11) **EP 1 870 450 A1**
(43) Date of publication of application: **26.12.2007**
(21) Application number: 06012781.8
(22) Date of filing: 21.06.2006
(51) Int. Cl.: C12N 5/00

(54) **Process for the culturing of E1-immortalized HER cells**

(71) Applicant: DSM IP Assets B.V., 6411 TE Heerlen (NL)
(72) Inventor: The designation of the inventor has not yet been filed
(74) Representative: Heijman, Renske Harriët

(57) **Abstract**

The invention relates to a process for the culturing of E1-immortalized HER cells, preferably PER.C6 cells in a cell culture medium, wherein the cell culture medium comprises fructose, in particular D-fructose. In a preferred embodiment of the invention, fructose is fed to the cell culture medium and the cell culture medium comprises glucose.

## Description

The invention relates to a process for the culturing of E1-immortalized HER cells in a cell culture medium. Also, the invention relates to a process for the production of a biological substance by culturing of E1-immortalized HER cells that produce the biological substance.

Such a process is for instance known from WO 2004/099396 wherein PER.C6 cells expressing a human IgG1 were grown in a batch process in EX-CELL^{™} VPRO medium (JRH Biosciences, Inc., USA) supplemented with 6mM L-glutamine or were grown in a fed-batch process in EX-CELL^{™} VPRO medium supplemented with 6mM L-glutamine to which a feed of the same medium (also supplemented with 6mM glutamine) or medium supplements, such as glucose, glutamine, amino acid, was added. The EX-CELL^{™} VPRO medium contains 30mM glucose.

In general, it appears advantageous if cell culture processes can be prolonged by improving viability of the cells in order to improve yields of products produced by the cell cultures. It is an object of the present invention to provide processes for culturing E1-immortalized HER cells wherein the viability of these cells is prolonged.

This object is achieved by culturing the E1-immortalized HER cells in a cell culture medium comprising fructose.

It has surprisingly been found that by culturing the cells in medium containing fructose instead of glucose, the viability of the E1-immortalized HER cells, in particular of PER.C6 cells is increased significantly. Furthermore, the culture time of the batch or fed-batch culture is prolonged significantly. This is surprising, since other carbohydrates, such as galactose or mannose do not have this effect.

Fructose, as used in the invention can be used in any form, for example in pure form, for example as pure D-fructose or as part of a composition. It may for example also be added in the form of a precursor of fructose, such as for example sucrose, which is a dissacharide of glucose and fructose. Fructose may also be used in the form of a fructose derivative.

An additional advantage of the process according to the invention is that the total cell number may be increased, hence increasing the total amount of biological substance produced (i.e. total yield). Another advantage of the addition of fructose as compared to glucose is that less lactate is produced.

The process of the present invention may for example be performed as a batch culture, as a fed-batch culture, as a continuous culture or as a perfusion culture.

Preferably, in the process of the present invention, the E1-immortalized HER cells are in a suspension culture.

Primary human embryonic retina (HER) cells can be isolated from fetuses (Byrd P, Brown KW, Gallimore PH. 1982. Malignant transformation of human embryo retinoblasts by cloned adenovirus 12 DNA. Nature 298: 69-71, Byrd PJ, Grand RJA, Gallimore PH. 1988. Differential transformation of primary human embryo retinal cells by adenovirus E1 regions and combinations of E1A + ras. Oncogene 2: 477-484). Primary cells will die upon culturing for several passages. E1-immortalized HER cells for the purpose of the present invention are derived from primary HER cells by expressing DNA encoding adenoviral E1A and E1B proteins therein, to obtain immortalized cells. Such immortalized cells can be cultured for more than 100 passages. Methods to obtain E1-immortalized HER cells have for instance been described in US patent 5,994,128, in Byrd P, Brown KW, Gallimore PH. 1982. Malignant transformation of human embryo retinoblasts by cloned adenovirus 12 DNA. Nature 298: 69-71, Byrd PJ, Grand RJA, Gallimore PH. 1988. Differential transformation of primary human embryo retinal cells by adenovirus E1 regions and combinations of E1A + ras. Oncogene 2: 477-484, and Gallimore, P.H., Grand, R.J.A. and Byrd, P.J. (1986). Transformation of human embryo retinoblasts with simian virus 40, adenovirus and ras oncogenes. AntiCancer Res. 6, p499-508. For instance, immortalized HER cells, including PER.C1, PER.C3, PER.C4, PER.C5, PER.C6, PER.C8 and PER.C9 cells, were generated by transfection of primary HER cells using a plasmid that contained the adenovirus serotype 5 (Ad5) E1A- and E1B-coding sequences (Ad5 nucleotides 459-3510) under the control of the human phosphoglycerate kinase ("PGK") promoter (see US patent 5,994,128).

In a preferred embodiment, the E1-immortalized HER cells in the process of the present invention are PER.C6 cells (see U.S. Patent 5,994,128). PER.C6 cells are exemplified by cells as deposited under ECACC No. 96022940 (see, e.g., U.S. Patent 5,994,128, EP 0833934 B1).

The PER.C6 cell line can be used for production of biological substances, such as E1-deleted adenovirus (see e.g. US patent 6,994,128; Nichols et al, 2002, Propagation of adenoviral vectors: use of PER.C6 cells. In: Curiel D, Douglas JT, editors. Adenoviral vectors for gene therapy. San Diego: Elsevier. p 129-167), other viruses (see e.g. WO 01/38362), or recombinant proteins (see e.g. US patent 6,855,544; Yallop et al, 2005, PER.C6 cells for the manufacture of biopharmaceutical proteins, Modern Biopharmaceuticals: Design, Development and Optimization, 4 Volumes, 779-807, Jörg Knäblein (Editor)). As such, PER.C6 cells are well documented for use in these processes, and the skilled person knows how to use these cells for the production of biological substances, such as adenovirus, other virus and proteins, by well documented methods as disclosed in the teachings of the referenced materials. Further, the cells are well documented from a regulatory point of view (see e.g. Nichols et al, supra). In addition, the cells are capable of growth in large scale bioreactors, can be cultured easily as adherent cells or in suspension and can be cultured in culture media that is free of animal components such as serum (see e.g. Yallop et al, supra). Each of these features contributes to the suitability of PER.C6 cells for the production of biological substances that can be used as an active pharmaceutical ingredient in a pharmaceutical preparation, for example for therapeutic and/or prophylactic use in humans.

Fructose may be added to the medium in several ways, for example fructose may be added to the cell culture medium before the cells are inoculated in the cell culture medium.. For example, the amount of fructose present in the cell culture medium is typically between 0.5mM and 60mM, more typically between 15mM and 50mM, in particular between 20mM and 40mM. Upon the teachings of the present disclosure, the optimal amount of fructose in the cell culture medium can easily be determined by the person skilled in the art through routine experimentation.

In certain embodiments, fructose is fed to the cell culture medium comprising the cells. The amount of fructose in the feed and the feed rate, is preferably chosen such that the amount of fructose in the cell culture medium is optimal. For example, the amount of fructose in the feed and the feed rate may be chosen such that the amount of fructose present in the cell culture medium is preferably between 0.5mM and 40mM, more preferably between 2mM and 30mM, in particular between 5mM and 20mM. The feed may be continuous or discontinuous (bolus).

The way in which fructose is added to the cell culture medium is not limited in any way. For example, in the process of the present invention, it is possible to feed fructose to a cell culture medium already comprising fructose or glucose or a different carbohydrate, such as for example galactose or mannose or to feed glucose or a different carbohydrate, such as for example galactose or mannose to a cell culture medium comprising fructose. Also in these cases, the person skilled in the art, considering the teaching of the present disclosure, can easily determine through routine experimentation the optimal amounts of fructose (or different carbohydrate) to be present in the cell culture medium and/or to be fed to the cell culture medium.

In a preferred embodiment of the invention, fructose is fed to a cell culture medium, wherein to the cell culture medium comprises glucose before inoculation of the cell culture medium with the cells.

In certain embodiments, the feed comprising fructose comprises glucose in an amount no more than 0.5 mM, more preferably no more than 0.2mM, most preferably the feed comprising fructose does not comprise glucose.

The optimal amount of glucose to be present in the cell culture medium before inoculation of the cells in the preferred embodiment of the invention can easily be determined by the person skilled in the art, upon the teaching of the present disclosure, through routine experimentation. In certain embodiments, the glucose concentration in the cell culture medium before inoculation of the cell culture medium with the cells is between 5mM and 60mM, for instance between 15mM and 50mM, in particular between 20mM and 40mM. Preferably, in this preferred embodiment of the invention, the fructose concentration in the cell culture medium, before inoculation of the cell culture medium is less than 40mM, more preferably less than 20mM, most preferably 10mM.

Besides fructose, the cell culture medium in the process of the present invention may further comprise components usually present in a cell culture medium. For example the cell culture medium may comprise salts and/or amino acids and/or vitamins and/or lipids and/or detergents and/or buffers and/or growth factors and/or hormones and/or cytokines and/or trace elements. Examples of salts include magnesium salts, for example MgCl₂.6H₂O, MgSO₄ and MgSO₄.7H₂O iron salts, for example FeSO₄.7H₂O, potassium salts, for example KH₂PO₄, KCl; sodium salts, for example NaH₂PO₄, Na₂HPO₄ and calcium salts, for example CaCl₂.2H₂O. Examples of amino acids include all known proteinogenic amino acids, for example hystidine, glutamine, threonine, serine, methionine. Preferably, at least glutamine or glutamic acid are added (for example before inoculation of the cell culture medium with the cells or fed to the cells during the culturing or a combination of both) in the cell culture medium, more preferably glutamic acid is present in the cell culture medium. Preferably glutamine and/or glutamic acid are present in a concentration between 2mM and 15mM, more preferably in a concentration between 2mM and 10mM, in particular in a concentration between 4mM and 8mM. Examples of vitamins include: ascorbate, biotin, choline.Cl, myo-inositol, D-panthothenate, riboflavin. Examples of lipids include: fatty acids, for example linoleic acid and oleic acid; The cell culture medium may also comprise other components, for example soy peptone or ethanol amine. Examples of detergents include Tween® 80 and Pluronic® F68. An example of a buffer includes HEPES and Na₂CO₃. Examples of growth factors/hormones/cytokines include insulin-like growth factor (IGF), hydrocortisone and (recombinant) insulin. Examples of trace elements are known to the person skilled in the art and include Zn, Mg and Se.

A special embodiment is a process according to the invention wherein the E1-immortalized HER cells produce a biological substance. A biological substance may for instance be a recombinant adenovirus (see e.g. US Patent 5,994,128; Nichols et al, supra), a virus other than an adenovirus, such as for example an influenzavirus (see e.g. WO 01/38362), or a recombinant protein, such as for instance an antibody (see e.g. US patent 6,855,544; Yallop et al, supra; Jones et al, 2003, High-level expression of recombinant IgG in the human cell line PER.C6. Biotechnol Prog. 19, 163-168).

E1-immortalized HER cells that produce a biological substance are for instance E1-immortalized HER cells capable of expressing a gene encoding the biological substance. E1-immortalized HER cells capable of expressing a gene encoding the biological substance may for example be prepared by transfection of the cells with a plasmid containing the gene encoding the biological substance and gene encoding a suitable selection marker, for example a gene encoding a neomycine resistance (Neo marker gene). Stably transfected cells may then be selected by selection pressure, for example - in the case of a Neo marker gene - by culturing the transfected cells in the presence of G418 (genericin) and immediate screening of the cells for cells exhibiting high-level expression of the biological substance. Methods for preparing clones of E1-immortalized HER cells expressing a protein, and methods for culturing such cells to produce the protein, are well known to the skilled person, and can for instance be found in (US 6,855,544; Jones et al, supra; Yallop et al, supra).

Biological substances, which may be produced by E1-immortalized HER cells, for example by expressing a (recombinant) gene coding therefore are for example proteins, in particular receptors, enzymes, fusion proteins, blood proteins such as proteins from the blood coagulation cascade, multifunctional proteins such as for instance erythropoietin, virus or bacterial proteins for instance for use in vaccines; immunoglobulins such as antibodies, for example IgG or IgM, and the like; Preferably, the biological substances such as proteins or vaccines produced by PER.C6 can be used as an active ingredient in a pharmaceutical preparation.

Within the framework of the present invention, with pharmaceutical preparation is meant any preparation, which can be used as a medicine, in particular as a medicine in humans. Such a medicine may for example be used for diagnosis, or for prophylactic purpose such as for instance a vaccine, and/or for therapeutic purpose, such as for instance an enzyme or protein for which a patient is deficient, or an antibody to kill undesired cells. A pharmaceutical preparation may further contain a pharmaceutically acceptable carrier or excipient, examples of which are well known to the person skilled in the art.

Examples of proteins that can be used as an active ingredient in pharmaceutical preparations (with the brand name between brackets) include Tenecteplase (TN Kase^{™}), (recombinant) antihemophilic factor (ReFacto^{™}), lymphoblastoid Interferon α-n1 (Wellferon^{™}), (recombinant) Coagulation factor (NovoSeven^{™}), Etanercept, (Enbrel^{™}), Trastuzumab (Herceptin^{™}), Infliximab (Remicade^{™}), Palivizumab (Synagis^{™}), Basiliximab (Simulect^{™}), Daclizumab (Zenapaz^{™}), Rituximab (Rituxan^{™}), (recombinant) Coagulation factor IX (Benefix^{™}) and Interferon β-1a (Avonex^{™}).

Examples of vaccines that can be used as an active ingredient in pharmaceutical preparation include isolated protein antigens, examples of which include but are not limited to live, oral, tetravalent Rotavirus vaccine (RotaShield^{™}), rabies vaccine (RanAvert^{™}), influenza vaccines and inactivated hepatitis A vaccine (VAQTA^{™}).

The pH, temperature, dissolved oxygen concentration and osmolarity of the cell culture medium are in principle not critical and depend on the type of cell chosen. Preferably, the pH, temperature, dissolved oxygen concentration and osmolarity are chosen such that it is optimal for the growth and productivity of the cells. The person skilled in the art knows how to find the optimal pH, temperature, dissolved oxygen concentration and osmolarity for the culture (see. e.g. WO 2004/099396). Preferably, for the process of the invention, the pH is chosen between 6.6 and 7.6 and/or the temperature is chosen between 30 and 39°C and/or the osmolarity is chosen between 260 and 400mOsm/kg.

For production of biological substances according to the invention, in particular if the biological substances are to be used as an active ingredient in pharmaceutical preparations, serum free media are preferred to media containing a serum source. The reason for this is that serum source media may be contaminated with viruses, present the risk of prionic infections, and can create a major obstacle in the downstream processing of the biopharmaceutical product (i.e. the further purification of the biological substance from the cell culture medium). Therefore the process of the invention is preferably performed in a cell culture medium that does not comprise serum from an animal, including human, source. Since compounds from a mammalian source also present an infection risk, preferably the cell culture medium is mammalian source free (i.e. the cell culture medium does not comprise serum or components from a mammalian source). More preferably the cell culture medium is animal source free (i.e. the cell culture medium does not comprise serum or components from an animal, including human, source. Examples of serum free media that can be used for the culturing of PER.C6 cells include commercially available media, such as for instance EX Cell^{™} VPRO medium (JRH Biosciences, catalog number 14561), HyQ^{®} CDM4Retino^{™} (HyClone), IS ProVec CD (Irvine scientific), 293-SFM II (invitrogen).

In preferred embodiments, the biological substance produced in the process of the present invention is harvested from the cells or from the culture medium or from both. The biological substance(s) produced in the process of the present invention can be further purified from the cell culture medium in so-called downstream processing, using methods dependent on the biological substance, which methods are as such well known to the skilled person. Downstream processing usually comprises several purification steps in varying combinations and order. Examples of purification steps in the downstream processing are separation steps (e.g. by affinity chromatography and/or ion exchange chromatography), steps for the concentration of the biological substance (e.g. by ultrafiltration or diafiltration), steps to exchange buffers and/or steps to remove or inactivate viruses (e.g. by virusfiltration, pH shift or solvent detergent treatment).

The invention will now be elucidated by way of the following examples, without however being limited thereto.

### Examples

### Experimental setup

In the present study, experiments were setup to investigate the effect of different carbon source on PER.C6 growth and production of E1-immortalized HER cells, exemplified in these experiments by PER.C6 cells. Media were prepared using EX-CELL™ VPRO medium (JRH Bioscience, U.S.A.) without glucose as a basal media. 20X glutamine (Gibco, U.S.A.) was added to obtain 6mM of glutamine in the medium and D-glucose, D-galactose, D-fructose and D-mannose (all from Merck, Germany) were added respectively to obtain four media at a concentration of 30mM. A PER.C6 clone expressing a recombinant antibody (see WO 2004/099396) was used in all studies. Cells were cultured in 500ml ventilated shake flasks (Corning, U.S.A.) with 75ml working volume, in a controlled incubator with 36.5°C, 5% CO₂ and a stirrer at a stirring speed of 100rpm.

Cultures were sampled every other day for viable cell count and the cell viability with Cedex cell counter (Innovatis, Germany) and for metabolic (glucose, lactate, glutamine, glutamate and NH4) and ion (Na and K) profile with BioProfile 400 (Nova Biomedical, U.S.A.). At the end of the culture, when cell viability dropped below 50%, antibody concentration was measured using Protein A column.

### Results

Results from the present study that the culture with fructose as carbon source had an enhanced growth and a slower death rate, resulting in a prolonged culture (Table 1 & 2) if compared to the use of galactose, mannose or glucose as a carbohydrate source. Also the culture with fructose as carbohydrate source showed minimal lactate production (Table 3). Total antibody production was also increased in the fructose culture, achieving 344mg/L at the end of the culture, compared to glucose culture, 208mg/L, galactose culture, 101 mg/L and mannose culture, 211 mg/L (Table 4).

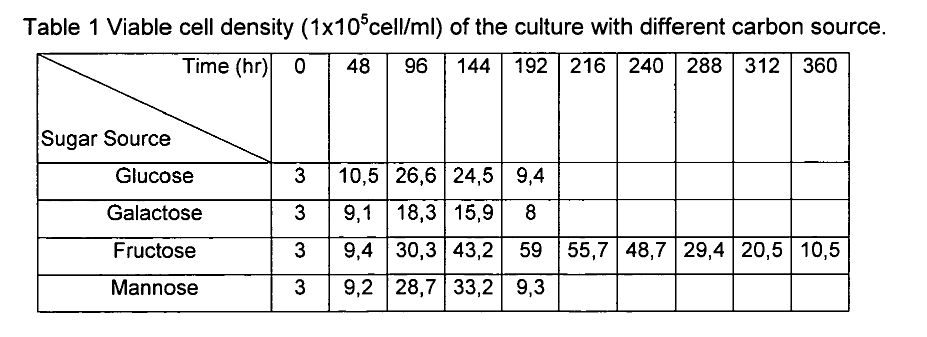

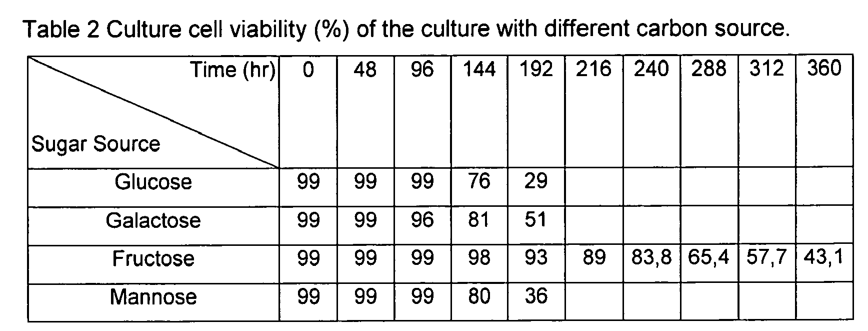

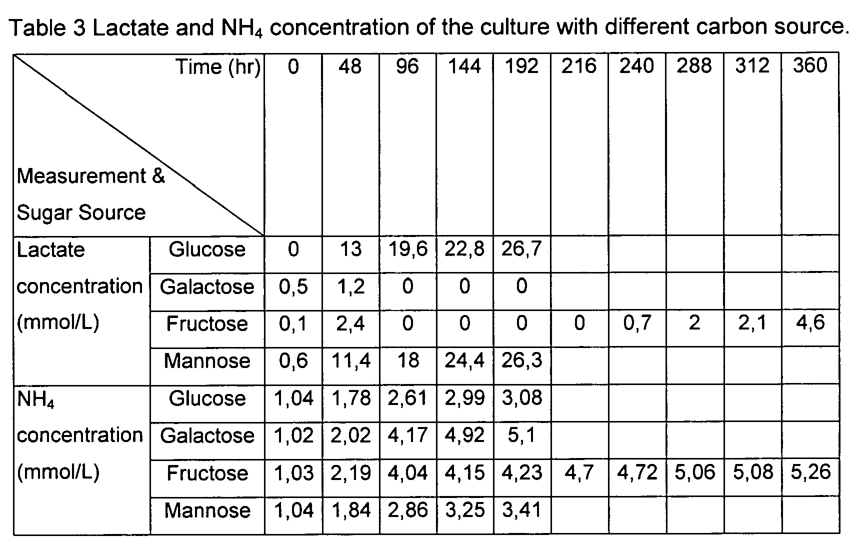

**Table 4 Final Antibody concentration of the culture with different carbon source.**

| Sugar Source | Total Antibody Production (mg/L) |
|---|---|
| Fructose | 344 |
| Glucose | 208 |
| Galactose | 101 |
| Mannose | 211 |

### Example 2 Effect of feeding of fructose

Experiments were setup to investigate the effect of using fructose as feed on cell growth and production. Media were prepared using EX-CELL™ VPRO medium (JRH) without glucose as a basal media. 20X glutamine (Gibco, U.S.A.) was added to obtain 6mM of glutamine in the medium. Glucose and fructose (Merck, Germany) were added respectively to obtain medium at a concentration specified in Table 5. A feeding solution of fructose was prepared at 2.5M solutions and added on day 6 to achieve the concentration also specified in Table 5. A PER.C6 clone expressing a recombinant antibody (see WO 2004/099396) was used in all studies. Cells were cultured in 500ml ventilated shake flasks (Corning, U.S.A.) with 75ml working volume, in a controlled incubator with 36.5°C, 10% CO₂ and a stirrer at a stirring speed of 200rpm.

**Table 5 Initial glucose and fructose concentration and feeding scheme in different cultures.**

| Culture | Initial glucose concentration (mM) | Initial fructose concentration (mM) | Fructose feed on day 6 (mM) | Glucose feed on day 6 (mM) |
|---|---|---|---|---|
| 1 | 0 | 30 | 0 | 0 |
| 2 | 20 | 0 | 20 | 0 |
| 3 | 0 | 20 | 20 | 0 |

Cultures were sampled every other day. The viable cell count and the cell viability were determined using a Cedex cell counter. The lactate concentration was determined using BioProfile 400 (NovaBiomedical, U.S.A.). The antibody concentration was determined with a Protein A column. Cultures were terminated when cell viability dropped below 70%. Results of this experiment are shown in table 6 below.

**Table 6 Maximum viable cell density, culture duration, final product and lactate concentration in the culture.**

| Culture | Maximum viable cell density (x10⁶ cell/ml) | Culture duration (days) | Final product concentration (µg/ml) | Final lactate concentration (mmol/L) |
|---|---|---|---|---|
| 1 | 5.81 | 13 | 296 | 1.4 |
| 2 | 6.65 | 11 | 337 | 16.9 |
| 3 | 5.44 | 13 | 225 | 7.6 |

As can be seen from table 6, the glucose culture to which fructose was fed had an enhanced growth, achieving a higher maximal viable cell density compared with other cultures. Total antibody production was also increased in the glucose culture with fructose feed, achieving 337µg/ml at the end of the culture, compared to fructose culture of 296µg/ml, and fructose with fructose feed of 225µg/ml.

## Claims

1. Process for the culturing of E1-immortalized HER cells, preferably PER.C6 cells in a cell culture medium, wherein the cell culture medium comprises fructose.

2. Process according to claim 1, wherein fructose is fed to the cell culture medium.

3. Process according to claim 1 or claim 2, wherein fructose is fed to a cell culture medium, wherein the cell culture medium comprises glucose.

4. Process according to claim 2 or claim 3, wherein fructose is fed to the culture medium, such that the fructose concentration in the culture medium is between 0.5mM and 40mM.

5. Process according to any one of claims 1-4, wherein the cell culture medium further comprises and/or is supplemented with glutamine or glutamic acid.

6. Process according to any one of claims 1-5, wherein the E1-immortalized HER cells produce a biological substance, wherein optionally the biological substance is harvested from the cells and/or from the culture medium.

7. Process according to claim 6, wherein the biological substance is a protein.

8. Process according to claim 7, wherein the protein is an antibody.

9. Process according to any one of claims 1-8, wherein the cell culture medium does not comprise serum from a human or an animal source.

10. Process according to any one of claims 1-9, wherein the cell culture medium does not comprise components from an animal source.
